(19) Europäisches Patentamt
European Patent Office
Office européen des brevets

(11) Veröffentlichungsnummer: **0 007 053**
**B1**

(12) # EUROPÄISCHE PATENTSCHRIFT

(45) Veröffentlichungstag der Patentschrift: **03.02.82**

(21) Anmeldenummer: **79102222.1**

(22) Anmeldetag: **02.07.79**

(51) Int. Cl.³: **C 07 F 9/65**, A 01 N 57/16 //C07D239/34

(54) **2-Cyclopropyl-pyrimidin(4)yl-(thiono)(thiol)-phosphor-(phosphon)-säureester, Verfahren zu ihrer Herstellung und ihre Verwendung als Insektizide und Akarizide.**

(30) Priorität: **13.07.78 DE 2830766**

(43) Veröffentlichungstag der Anmeldung:
**23.01.80 Patentblatt 80/2**

(45) Bekanntmachung des Hinweises auf die Patenterteilung:
**03.02.82 Patentblatt 82/5**

(84) Benannte Vertragsstaaten:
**AT BE CH DE FR GB IT NL SE**

(56) Entgegenhaltungen:
**EP - A - 0 000 887**
**FR - A - 1 063 067**
**FR - A - 2 378 791**
**US - A - 4 012 506**

(73) Patentinhaber: **BAYER AG**
**Zentralbereich Patente, Marken und Lizenzen**
**D-5090 Leverkusen 1, Bayerwerk (DE)**

(72) Erfinder: **Maurer, Fritz, Dr.**
**Roeberstrasse 8**
**D-5600 Wuppertal 1 (DE)**
Erfinder: **Schröder, Rolf, Dr.**
**Pahlkestrasse 17**
**D-5600 Wuppertal 1 (DE)**
Erfinder: **Hammann, Ingeborg, Dr.**
**Belfortstrasse 9**
**D-5000 Köln 1 (DE)**
Erfinder: **Stendel, Wilhelm, Dr.**
**In den Birken 55**
**D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 007 053 B1

Courier Press, Leamington Spa, England.

2-Cyclopropyl-pyrimidin(4)yl-(thiono)(thiol)-phosphor(phosphon-säureester, Verfahren zu ihrer Herstellung und ihre Verwendung als Insektizide und Akarizide

Die Erfindung betrifft neue 2-Cyclopropyl-pyrimidin(4)yl-(thiono)(thiol)-phosphor(phosphon)-säureester, ein Verfahren zu ihrer Herstellung sowie ihre Verwendung als Insektizide und Akarizide.

Es ist bekannt, daß bestimmte Pyrimidinyl-thionophosphorsäureester, wie z.B. O,O-Diäthyl-O-(2-methylthio-6-methyl-pyrimidin(4)yl-thionophosphorsäureester und der O,O-Diäthyl-O-(2-cyclopropyl-4-methylpyrimidyl-6)-thio-phosphorsäureester insektizide und akarizide Eigenschaften aufweisen (vgl. DE—PS 910 652 and US—PS 4 012 506).

Die Wirkung dieser Verbindungen ist jedoch, insbesondere bei niedrigen Aufwandmengen und Konzentrationen, nicht immer zufriedenstellend.

Es wurden nun die neuen 2-Cyclopropyl-pyrimidin(4)yl-(thiono)(thiol)-phosphor(phosphon)-säureester der Formel I

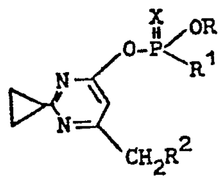

(I)

in welcher

R für Alkyl mit 1 bis 5 Kohlenstoffatomen steht,

$R^1$ für Alkyl, Alkoxy oder Alkylthio mit 1 bis 5 Kohlenstoffatomen oder Phenyl steht,

$R^2$ für Alkoxy oder Alkylthio mit 1 bis 5 Kohlenstoffatomen steht und

X für Sauerstoff oder Schwefel steht,

gefunden.

Die neuen Verbindungen zeichnen sich durch hohe Wirksamkeit bei der Schädlingsbekämpfung, insbesondere durch hohe insektizide und akarizide Wirksamkeit aus.

Weiter wurde gefunden, daß man die neuen 2-Cyclopropyl-pyrimidin(4)yl-(thiono)(thiol)-phosphor(phosphon)-säureester der Formel (I) erhält, wenn man (Thiono)(Thiol) Phosphor(phosphon)-säureester-halogenide der Formel II

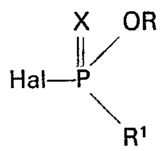

(II)

in welcher

R, $R^1$ und X die oben angegebene Bedeutung haben und

Hal für Chlor oder Brom steht,

mit 2-Cyclopropyl-4-hydroxy-pyrimidinen der Formel III

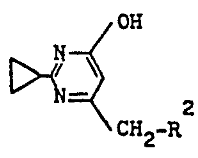

(III)

in welcher

$R^2$ die oben angegebene Bedeutung hat,

gegebenenfalls in Gegenwart eines Säureakzeptors und gegebenenfalls in Gegenwart eines Verdünnungsmittels umsetzt.

Überraschenderweise zeigen die erfindungsgemäßen 2-Cyclopropyl-pyrimidin(4)yl-(thiono)(thiol)-phosphor(phosphon)-säureester eine bessere Wirksamkeit bei der Schädlingsbekämpfung, insbesondere eine bessere insektizide und akarizide Wirkung als die entsprechenden aus dem Stand der Technik bekannten Verbindungen analoger Konstitution und gleicher Wirkungsrichtung. Die Produkte gemäß vorliegender Erfindung stellen somit eine wertvolle Bereicherung der Technik dar. Verwendet man beispielsweise O-n-Propyl-n-propan-phosphonsäureesterchlorid und 2-Cyclopropyl-6-äthoxymethyl-4-hydroxy-pyrimidin als Ausgangsverbindungen, so kann die ablaufende Reaktion durch folgendes Formelschema skizziert werden:

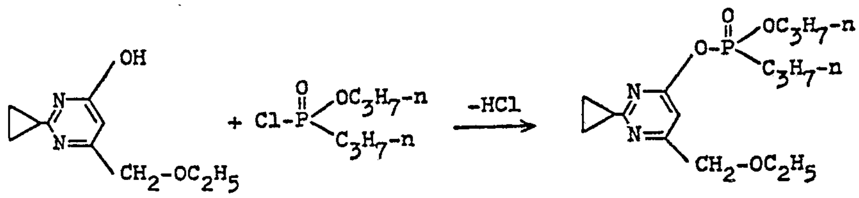


Die als Ausgangsstoffe zu verwendenden (Thiono)(Thiol)-Phosphor(phosphon)-säureesterhalogenide sind durch die Formel II definiert. Vorzugsweise stehen darin.

R für geradkettiges oder verzweigtes Alkyl mit 1 bis 5, insbesondere mit 1 bis 3 Kohlenstoffatomen.

$R^1$ für geradkettiges oder verzweigtes Alkyl mit 1 bis 5, insbesondere mit 1 bis 5 Kohlenstoffatomen, geradkettiges oder verzweigtes Alkoxy oder Alkylthio mit 1 bis 5, insbesondere mit 1 bis 3 Kohlenstoffatomen je Alkylrest oder für Phenyl,

X für Sauerstoff oder Schwefel und

Hal für Chlor.

Die Ausgangsstoffe der Formel II sind bekannte Verbindungen. Als Beispiele hierfür seien genannt: O-Methyl-, O-Äthyl-, O-n-Propyl-, O-iso-Propyl-methan-, -äthan-, -propan- und -phenylphosphonsäureesterchlorid sowie die entsprechenden Thionoanalogen, ferner O,O-Dimethyl-, O,O-Diäthyl-, O,O-Di-n-propyl-, O-Methyl-O-äthyl-, O-Methyl-O-n-propyl-, O-Methyl-O-iso-propyl-, O-Äthyl-O-n-propyl- und O-Äthyl-O-iso-propyl-phosphorsäurediesterchlorid sowie die entsprechenden Thionoanalogen, ferner O,S-Dimethyl-, O,S-Diäthyl-, O,S-Di-n-propyl-, O,S-Di-iso-propyl-, O-Methyl-S-äthyl-, O-Methyl-S-n-propyl-, O-Methyl-S-iso-propyl-, O-Äthyl-S-methyl-, O-Äthyl-S-n-propyl-, O-Äthyl-S-iso-propyl-, O-n-Propyl-S-methyl-, O-n-Propyl-S-äthyl-, O-n-Propyl-S-iso-propyl-, O-iso-Propyl-S-methyl-, O-iso-Propyl-S-äthyl- und O-iso-Propyl-S-n-propyl-thiolphosphorsäurediesterchlorid sowie die entsprechenden Thionoanalogen.

Die weiter als Ausgangsstoffe zu verwendenden 2-Cyclopropyl-4-hydroxy-pyrimidine sind durch Formel III definiert. Vorzugsweise steht darin

$R^2$ für geradkettiges oder verzweigtes Alkoxy oder Alkylthio mit 1 bis 5, insbesondere mit 1 bis 3 Kohlenstoffatomen je Alkylrest.

Die 2-Cyclopropyl-4-hydroxy-pyrimidine der Formel III sind neu. Man erhält diese Verbindungen durch Umsetzung der entsprechenden $\gamma$-Alkoxy- bzw. $\gamma$-Alkylthio-acetessigsäure-methyl- oder -äthylester mit Cyclopropancarbonsäureamidin-hydrochloriden bei Temperaturen zwischen —10 und 100°C, vorzugsweise zwischen 0 und 50°C, gegebenenfalls in Gegenwart eines Säureakzeptors wie z.B. Natriummethylat und gegebenenfalls in Gegenwart eines Verdünnungsmittels wie z.B. Methanol. Zur Aufarbeitung wird das Lösungsmittel im Vakuum abgezogen, der Rückstand in Eiswasser gelöst, mit Salzsäure pH 5 eingestellt und das sich abscheidende Produkt durch Vakuumfiltration isoliert.

Als Beispiele für die Verbindungen der Formel III seien im einzelnen genannt:

6-Methoxymethyl-, 6-Äthoxymethyl-, 6-n-Propoxymethyl- und 6-iso-Propoxymethyl-2-cyclopropyl-4-hydroxy-pyrimidin sowie 6-Methylthiomethyl-, 6-Äthylthiomethyl-, 6-n-Propylthiomethyl- und 6-iso-Propylthiomethyl-2-cyclopropyl-4-hydroxy-pyrimidin.

Das Verfahren zur Herstellung der erfindungsgemäßen 2-Cyclopropyl-pyrimidin(4)yl-(thiono)(thiol)-phosphor(phosphon)-säureester wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorokohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen —10 und 100°C, vorzugsweise bei +10 bis 80°C. Das erfindungsgemäße Verfahren wird im allgemeinen bei Normaldruck durchgeführt.

Zur Durchführung des erfindungsgemäßen Verfahrens werden auf 1 Mol (Thiono)(Thiol)phosphor(phosphon)-säureesterhalogenid II 1,0 bis 1,2 Mol 2-Cyclopropyl-4-hydroxy-pyrimidin III eingesetzt. Die Umsetzung wird im allgemeinen in einem der angegebenen Verdünnungsmittel in Gegenwart eines Säureakzeptors durchgeführt und das Reaktionsgemisch wird mehrere Stunden bei der erforderlichen Temperatur gerührt. Danach gibt man ein organisches Lösungsmittel, wie z.B. Toluol, dazu und arbeitet die organische Phase wie üblich durch Waschen, Trocknen und Abdestillieren des Lösungsmittels auf.

Die neuen Verbindungen fallen in Form von Ölen an, die sich zum Teil nicht unzersetzt destillieren

lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen 2-Cyclopropyl-pyrimidin(4)yl-(thiono)(thiol)-phosphor(phosphonsäureester zeichnen sich insbesondere durch eine hervorragende insektizide und akarizide Wirksamkeit gegenüber Pflanzen-, Hygiene-, Vorratsschädlingen und Ektoparasiten aus. Sie besitzen eine sehr gute Wirkung gegen saugende und fressende Insekten und Milben.

Aus diesem Grunde können die erfindungsgemäßen Verbindungen mit Erfolg im Pflanzenschutz sowie auf dem Hygiene-, Vorratsschutz- und veterinär-medizinischen Sektor als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophaus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta, domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis, gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euoxa spp., Feltia spp. Earias insulana, Heliothis spp., Laphygma exigua, Mamestra brassicae, Panolis flammea, Prodenia litura, Spodoptera spp., Trichoplusia ni, Carpocapsa, pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca, spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Certatitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia

praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsäzen, wie Räucherpatronen, -dosen, -spiralen, u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-metallphthalocyaninfarbstoffe und Spurennährstoffe wie Salze von eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

## Beispiel A

Plutella-Test

| | | |
|---|---|---|
| Lösungsmittel: | 3 Gewichtsteile | Aceton |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt,

solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 3, 4, 6, 7, 8, 9 und 10.

Beispiel B

Tetranychus-Test (resistent)

Lösungsmittel: 3 Gewichtsteile Aceton

Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeiren Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 2, 7, 8, 9 und 10.

Beispiel C

Test mit parasitierenden adulten Rinderzecken (Boophilus microplus rex.)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b. microplus res.) wreden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 3, 4, 6 und 10.

Herstellungsbeispiele

Beispiel 1: 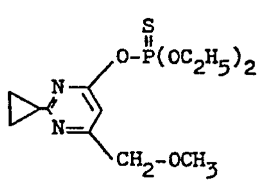

Ein Gemisch aus 150 ml Acetonitril, 9,9 g (55 mMol) 2-Cyclopropyl-6-methoxymethyl-4-hydroxy-pyrimidin und 8,4 g (60 mMol) Kaliumcarbonat wird eine Stunde bei 50°C gerührt. Anschließend kühlt man die Mischung auf Raumtemperatur ab und fügt 9,4 g (50 mMol) O,O-Diäthylthionophosphorsäurediesterchlorid hinzu. Nach vierstündigem Rühren bei 50—60°C wird die Reaktionslösung mit 200 ml Wasser und 300 ml Toluol geschüttelt, getrennt, die organische Phase über Magnesiumsulfat getrocknet und nach Filtration das Toluol am Rotationsverdampfer unter vermindertem Druck abgezogen. Es verbleiben 14 g (85% der Theorie) O,O-Diäthyl-O-(2-cyclopropyl-6-methoxymethyl-pyrimidin(4)yl)-thionophosphorsäureester in Form eine gelben Öles mit dem Brechungsindex $n_D^{20}$: 1,5483.

Analog Beispiel 1 können die folgenden Verbindungen der Formel I

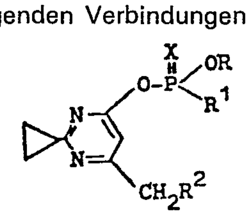 (I)

hergestellt werden:

| Beispiel Nr: | R | $R^1$ | $R^2$ | X | Ausbeute (% der Theorie) | Brechungsindex: |
|---|---|---|---|---|---|---|
| 2 | $C_2H_5$ | $C_2H_5$ | $OCH_3$ | S | 81 | $n_D^{20}$: 1,5239 |
| 3 | $C_2H_5$ | 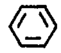 | $OCH_3$ | S | 91 | $n_D^{20}$: 1,5673 |
| 4 | $CH_3$ | $OCH_3$ | $OCH_3$ | S | 81 | $n_D^{20}$: 1,5302 |
| 5 | $C_2H_5$ | $OC_2H_5$ | $OCH_3$ | O | | $n_D^{20}$: 1,5368 |
| 6 | $C_2H_5$ | $SC_3H_7$-n | $OCH_3$ | S | 90 | $n_D^{20}$: 1,5360 |
| 7 | $C_2H_5$ | $OC_2H_5$ | $SCH_3$ | S | 75 | $n_D^{24}$: 1,5401 |
| 8 | $C_2H_5$ | $C_2H_5$ | $SCH_3$ | S | 74 | $n_D^{24}$: 1,5608 |
| 9 | $C_3H_7$-iso | $CH_3$ | $OCH_3$ | S | 91 | $n_D^{20}$: 1,5258 |
| 10 | $C_2H_5$ | $CH_3$ | $OCH_3$ | S | 81 | $n_D^{20}$: 1,5371 |

Die als Ausgangsstoffe benötigten 2-Cyclopropyl-4-hydroxy-pyrimidine können wie folgt hergestellt werden:

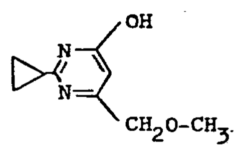

a)

Eine Lösung von 20 g (0,16 Mol) Cyclopropancarbonsäureamidinhydrochlorid und 21,9 g (0,15 Mol) $\gamma$-Methoxyacetssigsäuremethylester in 150 ml Methanol wird bei 5°C mit 63 g (0,3 Mol) Natriummethylat in Methanol versetzt, das Gemisch 3 Stunden bei Raumtemperatur gerührt und dann unter vermindertem Druck das Lösungsmittel abgezogen. Der Rückstand wird in 200 ml Eiswasser gelöst, unter Außenkühlung mit konzentrierter Salzsäure auf pH~5 gebracht, der Niederschlag abgesaugt und im Exsikkator über Phosphorpentoxid getrocknet. Man erhält 18 g (67% der Theorie) 2-Cyclopropyl-6-methoxy-methyl-4-hydroxy-pyrimidin in Form eines weißen Pulvers mit einem Schmelzpunkt von 178°C.

Analog wird hergestellt:

2-Cyclopropyl-6-methylmercaptomethyl-4-hydroxy-pyrimidin in 60%iger Ausbeute mit dem Schmelzpunkt von 150°C.

## Patentansprüche

1. 2-Cyclopropyl-pyrimidin(4)yl-(thiono)(thiol)-phosphor(phosphon)-säureester der Formel I

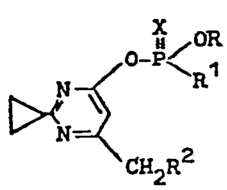

(I)

in der

R für Alkyl mit 1 bis 5 Kohlenstoffatomen steht,
$R^1$ für Alkyl, Alkoxy oder Alkylthio mit 1 bis 5 Kohlenstoffatomen oder Phenyl steht,
$R^2$ für Alkoxy oder Alkylthio mit 1 bis 5 Kohlenstoffatomen steht und
X für Sauerstoff oder Schwefel steht.

2. Verfahren zur Herstellung von 2-Cyclopropyl-pyrimidin-(4)yl-(thiono)(thiol)-phosphor(phosphon)-säureestern der Formel I

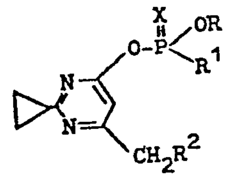

(I)

in der

R für Alkyl mit 1 bis 5 Kohlenstoffatomen steht,

$R^1$ für Alkyl, Alkoxy oder Alkylthio mit 1 bis 5 Kohlenstoffatomen oder Phenyl steht,

$R^2$ für Alkoxy oder Alkylthio mit 1 bis 5 Kohlenstoffatomen steht und

X für Sauerstoff oder Schwefel steht.

dadurch gekennzeichnet, daß man (Thiono)(Thiol)phosphor(phosphon)-säureester-halogenide der Formel II

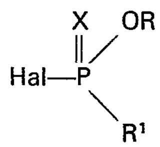

(II)

in der

R, $R^1$ und X die oben angegebene Bedeutung haben und

Hal für Chlor oder Brom steht,

mit 2-Cyclopropyl-4-hydroxy-pyrimidinen der Formel III

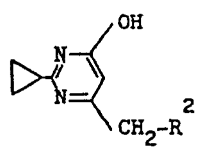

(III)

in der

$R^2$ die oben angegebene Bedeutung hat,

gegebenenfalls in Gegenwart eines Säureakzeptors und gegebenenfalls in Gegenwart eines Verdünnungsmittels umsetzt.

3. Schädlingsbekämpfungsmittel, gekennzeichnet durch einen Gehalt an Verbindungen der Formel I.

4. Verwendung von Verbindungen der Formel I zur Bekämpfung von Insekten und Milben.

5. Verfahren zur Herstellung von insektiziden und akariziden Mitteln, dadurch gekennzeichnet, daß man Verbindungen gemäß Formel I mit Streckmitteln und/oder oberflächenaktiven Mitteln mischt.

**Claims**

1. Cyclopropyl-pyrimidin-4-yl-(thiono)(thiol)-phosphoric(phosphonic) acid esters of the formula I

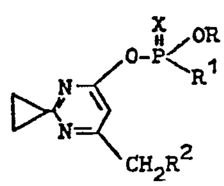

(I)

in which

R represents alkyl with 1 to 5 carbon atoms,

$R^1$ represents alkyl, alkoxy or alkylthio with 1 to 5 carbon atoms or phenyl,

$R^2$ represents alkoxy or alkylthio with 1 to 5 carbon atoms and

X represents oxygen or sulphur.

2. Process for the preparation of 2-cyclopropyl-pyrimidin-4-yl-(thiono)(thiol)-phosphoric-(phosphonic) acid esters of the formula I

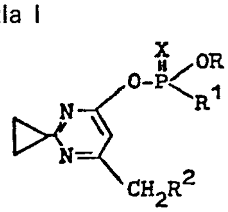

(I)

in which

R represents alkyl with 1 to 5 carbon atoms,

$R^1$ represents alkyl, alkoxy or alkylthio with 1 to 5 carbon atoms or phenyl,

$R^2$ represents alkoxy or alkylthio with 1 to 5 carbon atoms and

X represents oxygen or sulphur,

characterised in that (thiono)(thiol)-phosphoric(phosphonic) acid ester halides of the formula II

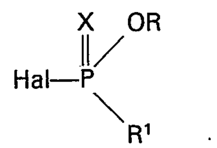

(II)

in which

R, $R^1$ and X have the meaning indicated above and

Hal represents chlorine or bromine,

are reacted with 2-cyclopropyl-4-hydroxy-pyrimidines of the formula III

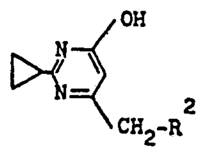

(III)

in which

$R^2$ has the meaning indicated above,

if appropriate in the presence of an acid acceptor and if appropriate in the presence of a diluent.

3. Agents for combating pests, characterised in that they contain compounds of the formula I.

4. Use of compounds of the formula I for combating insects and mites.

5. Process for the preparation of insecticidal and acaricidal agents, characterised in that compounds according to formula I are mixed with extenders and/or surface-active agents.

**Revendications**

1. Esters (thiono)(thiol)-phosphor(phosphon)iques de 2-cyclopropyl-pyrimidine(4)yle de formule

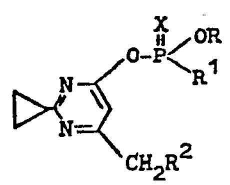

(I)

dans laquelle

R représente un alkyle en $C_1$—$C_5$,

$R^1$ représente un alkyle, un alcoxy ou un alkylthio en $C_1$—$C_5$ ou un phényle,

$R^2$ représente un alcoxy ou un alkylthio en $C_1$—$C_5$ et

X représente l'oxygène ou le sourfre.

2. Procédé pour la préparation d'esters (thiono)(thiol)-phosphor(phosphon)iques de 2-cyclopropyl-pyrimidine(4)yle de formule

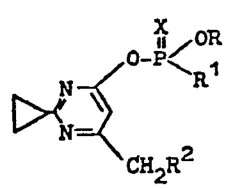

(I)

dans laquelle

R représente un alkyle en $C_1$—$C_5$,

$R^1$ représente un alkyle, un alcoxy ou un alkylthio en $C_1$—$C_5$ ou un phényle,

$R^2$ représente un alcoxy ou un alkylthio en $C_1$—$C_5$ et

X représente l'oxygène ou le soufre,

caractérisé en ce que l'on fait réagir des halogénures-esters (thiono)(thiol)phosphor(phosphon)iques de formule

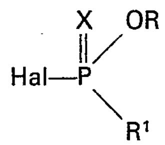

(II)

dans laquelle

R, $R^1$ et X ont la signification indiquée ci-dessus et

Hal représente le chlore ou le brome,

avec des 2-cyclopropyl-4-hydroxy-pyrimidines de formule

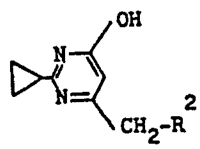

(III)

dans laquelle

$R^2$ a la signification indiquée ci-dessus,

éventuellement en présence d'un accepteur d'acide et éventuellement en présence d'un agent diluant.

3. Agents de lutte contre les parasites, caractérisés en ce qu'il contiennent des composés de formule I.

4. Utilisation de composés de formule I pour la lutte contre les insectes et les araignées.

5. Procédé pour la préparation d'agents insecticides et acaricides, caractérisé en ce que l'on mélange des composés de formule I avec des agents diluants et/ou des agents tensioactifs.